# FASCICULE DE BREVET EUROPEEN

(11) **EP 1 185 222 B1**
(45) Date de publication et mention de la délivrance du brevet: **09.01.2008**
(21) Numéro de dépôt: 99920909.1
(22) Date de dépôt: 21.05.1999
(51) Int. Cl.: A61F 9/007

(54) **SONDE BICANALICULAIRE POUR LE TRAITEMENT DU LARMOIEMENT DE L'OEIL**
BIKANALIKULÄRSONDE FÜR DIE BEHANDLUNG DES TRÄNENTRÄUFELNS BEI AUGEN
BI-CANALICULAR PROBE FOR THE TREATMENT OF LACRIMATION OF THE EYE

(43) Date de publication de la demande: 13.03.2002
(73) Titulaire: Bige, Pierre André Jacques, 83270 Saint-Cyr-sur-Mer (FR)
(72) Inventeur: Bige, Pierre André Jacques, 83270 Saint-Cyr-sur-Mer (FR)
(74) Mandataire: Bentz, Jean-Paul
(86) Numéro de dépôt international: PCT/FR1999/001213
(87) Numéro de publication internationale: WO 2000/071062

(56) Documents cités:
- WO-A-91/14406
- CH-A- 676 776
- FR-A- 2 735 697
- FR-A- 2 771 297
- GB-A- 2 273 243
- US-A- 4 305 395
- US-A- 4 753 637
- US-A- 5 052 998
- US-A- 5 318 513
- US-A- 5 437 625

## Description

La présente invention concerne une sonde bicanaliculaire destinée à traiter une anomalie d'irrigation lacrymale de l'oeil, et en particulier une anomalie provoquant un larmoiement de l'oeil.

Plus précisément, l'invention concerne une sonde bicanaliculaire pour le traitement d'une anomalie d'irrigation lacrymale de l'oeil cette sonde comprenant un élément filiforme en matériau biologiquement inerte qui, par introduction de la sonde, est sélectivement inséré dans les canalicules lacrymaux supérieur et inférieur de l'oeil et qui présente deux extrémités libres munies d'éléments terminaux respectifs sélectivement mis en place dans le sac lacrymal ou dans la fosse nasale.

On sait en effet que le liquide lacrymal s'écoule normalement, pour sa plus grande partie, par des méats supérieur et inférieur se trouvant dans le coin de l'oeil du côté du nez, et que ces méats communiquent, par les canalicules lacrymaux, avec le sac lacrymal. A partir du sac, le liquide lacrymal s'écoule ensuite dans la fosse nasale par le canal lacrymo-nasal.

Or, les voies lacrymales sont susceptibles de présenter plusieurs pathologies, qu'il est connu de corriger au moyen de sondes.

Le brevet US 5 318 513 décrit par exemple une sonde bicanaliculaire, du type précédemment défini, destinée à traiter un défaut d'irrigation lacrymale provoquant une sécheresse de l'oeil.

Dans cette sonde connue, les éléments terminaux sont constitués par des ballonnets qui assurent à la fois l'obturation de la jonction des canalicules lacrymaux au sac lacrymal, et l'immobilisation totale de la sonde dans ces canalicules.

Si l'enseignement du brevet US 5 318 513 peut éventuellement être mis à profit pour concevoir d'autres sondes destinées à traiter la sécheresse de l'oeil, il n'est en revanche d'aucune pertinence ni d'aucune utilité pour mettre au point une sonde bicanaliculaire destinée, comme c'est le cas de la sonde de l'invention, à éviter un larmoiement de l'oeil.

Cependant on a déjà proposé, également, des sondes pour traiter la sténose des voies lacrymales, c'est-à-dire une obstruction plus ou moins prononcée des canalicules et des méats lacrymaux, qui provoque précisément l'apparition d'un larmoiement.

C'est ainsi que l'on connaît déjà des sondes essentiellement constituées d'un élément filiforme en silicone qui revêt la forme d'un tube très fin et dont les deux extrémités sont chacune munies d'une aiguille.

Une aiguille est introduite par l'un des deux méats lacrymaux jusqu'au delà des fosses nasales en passant successivement dans un canalicule, le sac lacrymal et le canal lacrymonasal, et ensuite l'autre aiguille est introduite par l'autre méat jusqu'au delà des fosses nasales en passant successivement dans un deuxième canalicule puis dans le sac lacrymal et le canal lacrymonasal précités. Ensuite, les deux aiguilles sont séparées de l'élément filiforme tubulaire par sectionnement et les deux extrémités sectionnées de cet élément sont nouées au niveau des fosses nasales.

Une telle sonde présente des inconvénients en ce qu'elle nécessite une intubation jusqu'aux fosses nasales, ce qui n'est nullement nécessaire lorsqu'on se trouve en face d'une pathologie concernant les méats, les deux canalicules et/ou le canal d'union ou de jonction des deux canalicules aboutissant au sac lacrymal.

En outre, la mise en place d'une telle sonde est relativement délicate, traumatisante pour le patient et plus ou moins bien tolérée, sans parler du fait qu'elle doit être nécessairement implantée en hôpital ou en clinique.

Enfin, le noeud formé aux extrémités de l'élément filiforme doit être généralement fixé à la paroi du nez car il ne constitue pas sinon un moyen de retenue efficace. Cette fixation est traumatisante pour le patient et nécessite un geste supplémentaire pour le chirurgien.

Il arrive aussi que le canal lacrymonasal soit obstrué. Dans ce cas, il est connu de procéder à une opération appelée DACRYOCYSTORHINOSTOMIE ou DCR qui consiste à établir une dérivation, en ouvrant le sac lacrymal et en créant, par une ostéotomie, un canal d'écoulement direct des larmes dans la fosse nasale, sans passer par le canal lacrymonasal obstrué.

On réalise alors une ouverture en regard du sac lacrymal correspondant. La muqueuse de la paroi nasale ouverte est ensuite suturée avec la muqueuse du sac lacrymal. Les larmes s'écoulent directement vers la fosse nasale et non plus par le canal lacrymonasal.

Malheureusement, les parois muqueuses de l'ouverture ont généralement tendance, par prolifération cellulaire, à obstruer l'anastomose réalisée en regard du canal d'union ce qui empêche l'écoulement des larmes et ramène à la situation antérieure.

On connaît par ailleurs, grâce au document de brevet FR-2 735 697, une sonde bicanaliculaire comprenant un fil tubulaire sur lequel est formée au moins une excroissance élastique destinée à assurer une immobilisation de la sonde dans les canalicules, un brin du fil prolongeant chaque excroissance. A l'extrémité libre de chaque brin du fil, est fixé un mandrin métallique pour la mise en place -de la sonde.

La sonde est mise en place sur un patient de telle sorte que les excroissances sont placées dans une ouverture réalisée dans l'os de la fosse nasale, les brins du fil s'étendant dans la fosse nasale.

La présence d'au moins une excroissance dans l'ouverture de la fosse nasale permet d'éviter son obstruction pendant le temps nécessaire à la cicatrisation des muqueuses autour de l'ouverture.

Cependant, la présence des ces excroissances rend difficile la mise en place de la sonde dans les voies lacrymales. La taille des excroissances est donc nécessairement limitée et ces excroissances ne permettent pas de bien maintenir en place la sonde dans une ostéotomie dont le diamètre est d'au moins 4 mm et qui n'est souvent pas régulière. C'est pourquoi, il est encore nécessaire de réaliser un noeud avec les brins du fil s'étendant dans la fosse nasale et de le fixer à la paroi du nez, ce qui est traumatisant pour le patient.

La présente invention a donc pour but de remédier à ces problèmes en proposant une sonde bicanaliculaire qui présente un encombrement minimal et qui soit apte à rétablir le fonctionnement des méats, des deux canalicules, du canal d'union de ces deux canalicules au sac lacrymal, du sac lacrymal et/ou de la fosse nasale.

En outre, la sonde selon cette invention est très facile à mettre en place, est mieux tolérée par le patient et peut dans certains cas être mise en place au cabinet du praticien.

A cette fin, la sonde de l'invention, par ailleurs conforme à la définition générique qu'en donne le préambule ci-dessus, est essentiellement caractérisée en ce que les éléments terminaux sont constitués par des moyens de retenue flexibles se repliant vers l'élément filiforme lors de l'introduction de la sonde et s'épanouissant dans le sac lacrymal ou la fosse nasale pour former butée de retenue, et en ce qu'un jeu, autorisant un coulissement de la sonde mise en place dans les voies lacrymales, est ménagé entre les moyens de retenue et le sac lacrymal ou la fosse nasale, cette sonde permettant de remédier à un larmoiement de l'oeil, par maintien ou rétablissement du flux du liquide lacrymal.

Suivant une réalisation préférée, l'un au moins des moyens de retenue précité est constitué par une ancre comprenant au moins une branche flexible.

Chaque branche flexible de cette ancre est de préférence inclinée, à l'état de repos, vers l'élément filiforme.

L'un au moins des moyens de retenue peut aussi être constitué par un disque souple.

Dans un mode de réalisation possible, l'un au moins de ces moyens de retenue peut comprendre au moins deux branches.

Par ailleurs, chaque moyen de retenue peut avantageusement comporter une partie formant tige qui est reliée à l'élément filiforme et qui est munie d'un trou borgne apte à recevoir une tige ou bâtonnet permettant de la manoeuvrer.

Mais d'autres caractéristiques et avantages de l'invention apparaîtront mieux dans la description détaillée qui suit et se réfère aux dessins annexés, donnés uniquement à titre d'exemple, et dans lesquels:
- la figure 1 est une vue d'une sonde caniculonasale selon l'art antérieur, implantée sur un patient ;
- la figure 2 est une vue de côté, en élévation, d'une sonde bicanaliculaire selon l'invention ;
- la figure 3 est une vue agrandie en perspective de l'ancre à chaque extrémité de la sonde;
- la figure 4 est une vue de la sonde de l'invention mise en place sur un patient souffrant d'une obstruction des méats, des canalicules et/ou du canal de jonction des deux canalicules et
- la figure 5 est une vue de la sonde de l'invention mise en place sur un patient souffrant d'une obstruction du canal lacrymonasal et après DCR.

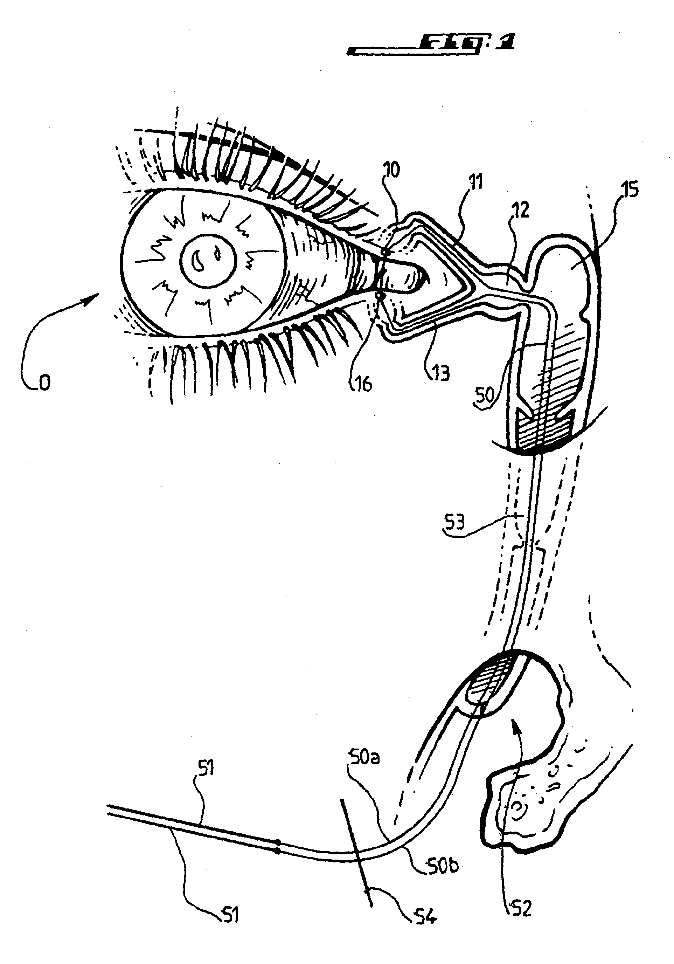

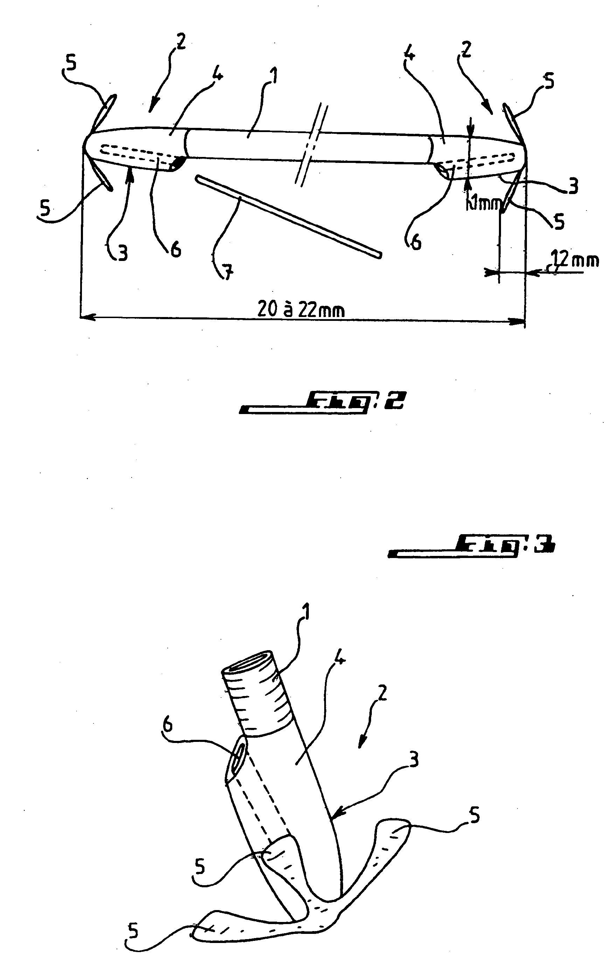

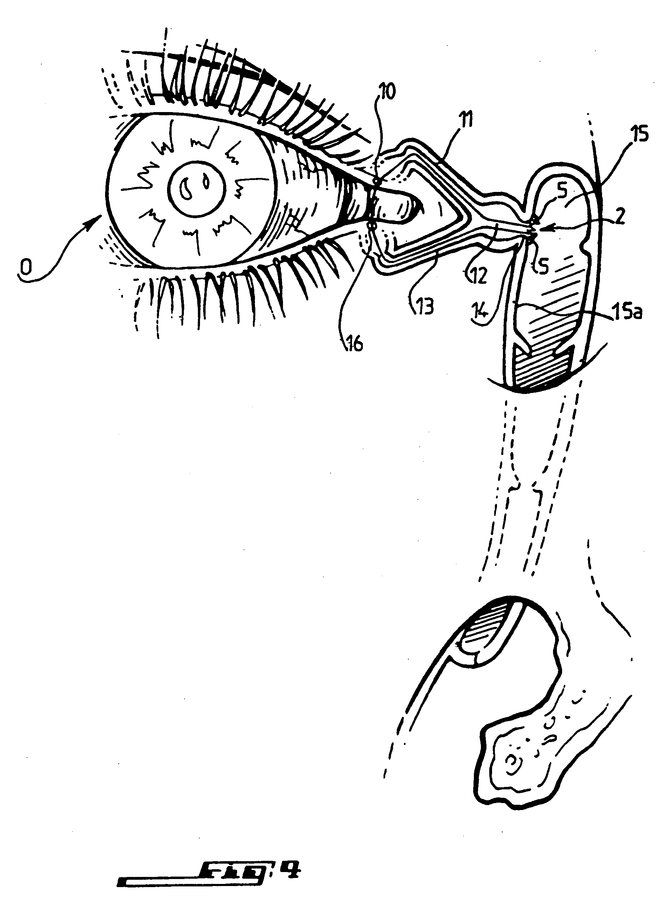

Suivant l'exemple représenté sur les figures 2 et 3, on voit qu'une sonde conforme au principe de l'invention est constituée par un élément filiforme 1 qui peut être un tube très fin en silicone ou autre matériau biologiquement inerte, dont les extrémités libres sont chacune munies d'un moyen de retenue 2.

Comme on le voit bien sur la figure 3, chaque moyen de retenue 2 forme une ancre 3 comportant une partie en forme de tige 4 qui porte à son extrémité plusieurs branches ou pattes flexibles 5, à savoir trois ou quatre branches suivant l'exemple représenté. La partie formant tige 4 de l'ancre 3 constitue les deux extrémités de l'élément filiforme ou tube fin en silicone 1.

Comme cela apparaît clairement sur les figures 2 et 3, à l'état de repos de la sonde, les branches flexibles 5 sont tournées du côté de l'élément filiforme 1.

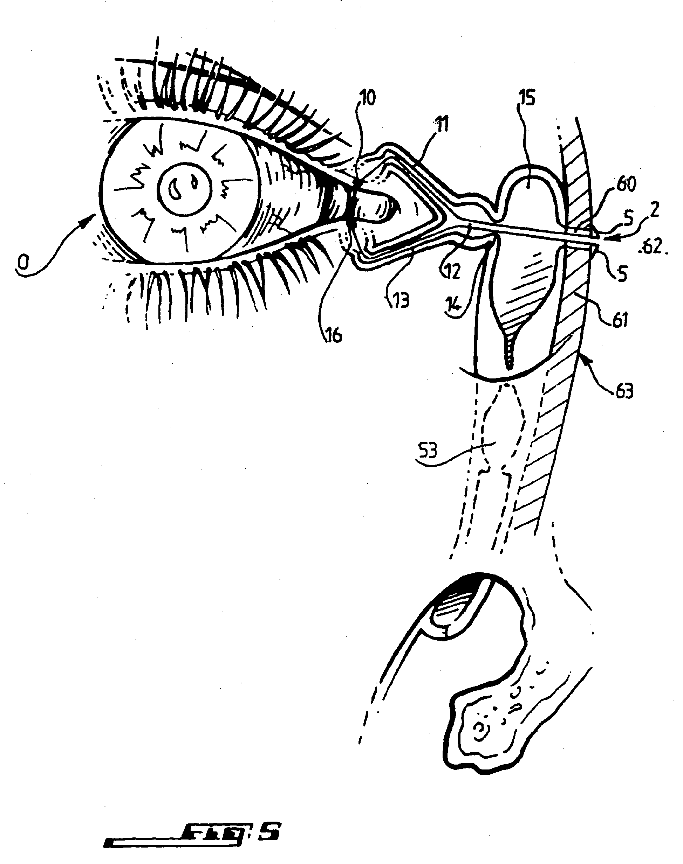

Chaque extrémité de cet élément 1, c'est-à-dire chaque partie en forme de tige 4 de l'ancre 3, est munie d'un trou borgne 6 apte à recevoir une tige ou analogue repérée en 7 sur la figure 2. Cette tige 7 permet de manoeuvrer l'extrémité en forme d'ancre de l'élément filiforme 1 pour réaliser la mise en place de la sonde comme on le décrira plus loin en référence à la figure 4.

Lorsque la sonde est utilisée pour traiter l'obstruction des méats lacrymaux, des canalicules et/ou du canal de jonction des deux canalicules, la longueur de l'élément filiforme 1 est comprise entre environ 15 et 30 mm. Suivant une réalisation préférée, cette longueur sera comprise entre 24 et 26 mm.

Quand la sonde est utilisée pour traiter une obstruction du canal lacrymonasal, la longueur de l'élément filiforme est comprise entre environ 38 et 60 mm et, de préférence, entre 44 et 46 mm.

Le diamètre du tube de silicone 1 pourra être de 0,64 mm, tandis que la tige 7 pourra avoir un diamètre de 0,3 mm environ.

Bien entendu, l'invention n'est pas limitée à la sonde qui vient d'être décrite. En particulier, l'ancre 3 pourrait ne comporter qu'une ou deux branches 5. De plus, le moyen de retenue pourrait également consister en un disque souple ou en un élément en T flexible, disposés avantageusement de façon à ce que les branches du T ou le disque soient sensiblement perpendiculaires à l'élément filiforme. Ces moyens de retenue ne sont pas illustrés sur les figures.

Le moyen de retenue pourrait alors être relié à l'élément filiforme 1 par une partie formant tige et comportant un trou borgne apte à recevoir un moyen de mise en place de la sonde sur le patient, tel que la tige 7 illustrée à la figure 2.

On décrira maintenant comment s'effectue la mise en place de la sonde qui vient d'être décrite, lorsqu'elle est utilisée pour traiter l'obstruction des méats lacrymaux, des canalicules et/ou du canal de jonction des deux canalicules. Ceci permettra d'expliciter ses avantages, en se référant à la figure 4.

La tige 7 étant enfoncée dans l'une des extrémités en forme d'ancre 3 de l'élément tubulaire en silicone 1, on introduira ainsi commodément une ancre 3 dans l'un des deux méats de l'oeil O, par exemple le méat supérieur 10. Il est à noter que lors de cette introduction, Les branches 5 de l'ancre s'effaceront facilement en se repliant contre le tube en silicone 1. Après franchissement du méat supérieur 10, l'ancre 3 poursuivra son trajet en passant dans le canalicule supérieur 11, puis dans le canal 12 d'union du canalicule supérieur 11 au canalicule inférieur 13 pour finalement franchir la jonction 14 formant valve entre le canal d'union 12 et le sac lacrymal 15. A ce point, c'est-à-dire dans le sac lacrymal 15, les branches flexibles 5 s'épanouiront naturellement et constitueront ainsi un moyen de retenue ou d'ancrage de la sonde à ce niveau, en formant une butée contre le sac lacrymal, et plus précisément contre la paroi interne 15a du sac après franchissement de la jonction 14.

On procède de la même façon que ci-dessus pour introduire l'autre ancre 3, c'est-à-dire l'autre extrémité de l'élément filiforme 1 dans le méat inférieur 16. Comme décrit précédemment, cette deuxième ancre passera successivement dans le canalicule inférieur 13 puis dans le canal d'union 12 pour finalement franchir la jonction 14, après quoi les branches flexibles 5 s'épanouiront naturellement.

Les moyens de retenue autres qu'une ancre se comporteront de la même façon, en se repliant contre l'élément filiforme 1 lors de l'introduction de la sonde et en s'épanouissant ensuite pour former une butée.

On comprend donc que les deux ancres 3 retiendront la sonde de l'invention par ses deux extrémités au niveau de la jonction 14 des canalicules lacrymaux 11, 13 au sac lacrymal 15.

On précisera ici que non seulement les branches flexibles des deux ancres 3 s'accrocheront au niveau de la jonction 14, mais ces branches 5 s'imbriqueront avantageusement entre elles ce qui assure une bonne retenue de la sonde.

Pour procéder à l'ablation de la sonde, il suffira d'exercer une légère traction sur elle, de sorte que les branches 5 des ancres 3, en raison de leur flexibilité, échapperont naturellement à la jonction 14.

On comprend donc de ce qui précède que la sonde selon cette invention ne nécessite qu'une intubation bicanaliculaire, c'est-à-dire une intubation minimale, contrairement aux sondes de l'art antérieur telles que celle montrée sur la figure 1, pour traiter une obstruction des méats, des deux canalicules et/ou du canal de jonction des deux canalicules.

On voit en effet sur cette figure, comme cela a été expliqué au début de la présente description, que cette sonde connue se compose d'un élément filiforme 50 en silicone dont les extrémités comportent chacune une aiguille 51 permettant l'introduction de la sonde par les méats supérieurs 10 et 11, et cela jusqu'aux fosses nasales 52 après passage dans les canalicules 11, 13, dans le canal d'union 12, dans le sac lacrymal 15 et dans le canal lacrymonasal 53. Une fois la sonde introduite comme on le voit sur la figure 1, l'élément filiforme 50 est sectionné comme on l'a montré schématiquement en 54, et on effectue un noeud avec les deux brins 50a, 50b de l'élément filiforme séparé des aiguilles 51, cela au niveau des fosses nasales 52 pour, en quelque sorte, fermer la boucle. Comme expliqué au début de la description, la mise en place d'une telle sonde est plus difficile, plus traumatisante pour le patient et moins bien tolérée.

Contrairement à cela, la sonde selon la présente invention est plus physiologique et plus facile d'utilisation. Elle ne nécessite pas d'intubation du canal lacrymonasal et peut être mise en place rapidement par un praticien en son cabinet.

On observera encore que la sonde selon cette invention permet de remédier aux plaies canaliculaires après suture, aux sténoses canaliculaires et du canal d'union, aux sténoses des méats lacrymaux, et aux défauts de positionnement des méats qui se produisent sous l'effet du renversement des paupières vers l'extérieur (ectropion) ou vers l'intérieur (entropion).

On va maintenant décrire la mise en place de la sonde selon l'invention, en cas d'obstruction du canal lacrymonasal 53, en référence à la figure 5.

Une ouverture 60 est pratiquée dans l'os 61 de la fosse nasale 62 pour constituer une anastomose entre les muqueuses du nez et du sac lacrymal suturées ensemble.

Il est nécessaire d'introduire une sonde par les méats 10 et 16 pour bien dégager un passage.

Comme expliqué précédemment en référence à la figure 4, une tige 7 étant enfoncée dans le trou borgne 6 à une extrémité de la sonde, une ancre 3 ou tout autre moyen de retenue est introduit par exemple par le méat supérieur 10.

Après franchissement du méat supérieur 10, l'ancre 3 poursuit son trajet en passant dans le canalicule supérieur 11, puis dans le canal 12 d'union du canalicule supérieur 11 au canalicule inférieur 13. L'ancre 3 franchit ensuite la jonction 14 entre le canal d'union et le sac lacrymal 15, puis le sac lacrymal 15 pour entrer dans l'ouverture 60 et ressortir dans la fosse nasale 62.

A ce point, les branches flexibles de l'ancre 3 s'épanouissent et constituent un moyen de retenue de la sonde sur la fosse nasale en formant une butée contre la paroi interne 63 de la fosse nasale, après franchissement de l'orifice 60.

L'autre extrémité de la sonde munie d'une ancre 3 est introduite de façon similaire dans le méat inférieur 16, et passe successivement dans le canalicule inférieur 13, dans le canal d'union, dans le sac lacrymal 15 et l'orifice 60, après quoi les branches 5 de l'ancre 3 s'épanouissent dans la fosse nasale 62 et forment une butée contre la paroi interne 63 de la fosse nasale, après franchissement de l'orifice 60, pour retenir la sonde.

La description qui précède vaut également pour tout autre moyen de retenue souple qui se replie contre l'élément filiforme 1 lors de l'introduction de la sonde et qui s'épanouit ensuite pour former une butée.

Par ailleurs, la sonde illustrée en référence à la figure 5 pour une fixation au niveau de la fosse nasale présente bien sûr une longueur plus importante que celle illustrée à la figure 4.

Une implantation au niveau de la fosse nasale doit être effectuée en hôpital ou clinique, notamment pour des raisons d'asepsie. La sonde selon l'invention présente l'avantage, par rapport aux sondes connues, d'être fixée à la fosse nasale sans qu'il soit nécessaire de réaliser un noeud avec les deux extrémités libres de la sonde puis de fixer ce noeud. Sa mise en place est donc simplifiée et moins traumatisante pour le patient.

Dans les deux modes d'implantation de la sonde selon l'invention qui viennent d'être décrits, la longueur de l'élément filiforme est choisie pour que les moyens de retenue ne soient pas directement en contact avec le sac lacrymal ou la fosse nasale après mise en place de la sonde.

En effet, les paupières et les voies lacrymales forment un ensemble dynamique, les voies lacrymales se contractant à chaque battement des paupières.

Ainsi, le jeu ménagé entre les moyens de retenue et le sac lacrymal ou la fosse nasale laisse à la sonde la possibilité de coulisser légèrement dans les voies lacrymales, ce qui évite toute lésion au niveau des méats lacrymaux et toute gêne pour le patient, les moyens de retenue venant temporairement en butée contre le sac lacrymal ou la fosse nasale pour maintenir la sonde dans les voies lacrymales.

Bien entendu, l'invention n'est nullement limitée au mode de réalisation de sonde décrit et illustré qui n'a été donné qu'à titre d'exemple.

A ce titre, par exemple, les extrémités en forme d'ancre de l'élément tubulaire en silicone pourront venir de matière ou de moulage avec élément filiforme 1 ou bien être rapportées et fixées sur cet élément tubulaire par tout moyen approprié. Il en est de même pour tout autre moyen de retenue.

## Revendications

1. Sonde bicanaliculaire pour le traitement d'une anomalie d'irrigation lacrymale de l'oeil, cette sonde comprenant un élément filiforme (1) en matériau biologiquement inerte qui, par introduction de la sonde, est sélectivement inséré dans les canalicules lacrymaux supérieur (11) et inférieur (13) de l'oeil et qui présente deux extrémités libres (4) munies d'éléments terminaux respectifs (2) sélectivement mis en place dans le sac lacrymal (15) ou dans la fosse nasale (62), **caractérisée en ce que** les éléments terminaux (2) sont constitués par des moyens de retenue flexibles se repliant vers l'élément filiforme (1) lors de l'introduction de la sonde et s'épanouissant dans le sac lacrymal (15) ou la fosse nasale (62) pour former butée de retenue, et **en ce qu'**un jeu, autorisant un coulissement de la sonde mise en place dans les voies lacrymales (11, 13), est ménagé entre les moyens de retenue (2) et le sac lacrymal (15) ou la fosse nasale (62), cette sonde permettant de remédier à un larmoiement de l'oeil par maintien ou rétablissement du flux du liquide lacrymal.

2. Sonde selon la revendication 1, **caractérisée en ce que** l'un au moins des moyens de retenue (2) est constitué par une ancre (3) comprenant au moins une branche flexible (5).

3. Sonde selon la revendication 2, **caractérisée en ce que** chaque branche flexible (5) de l'ancre (3), à l'état de repos, est inclinée vers l'élément filiforme (1).

4. Sonde selon la revendication 1, **caractérisée en ce que** l'un au moins des moyens de retenue (2) est constitué par un disque souple.

5. Sonde selon l'une quelconque des revendications 1 à 3, **caractérisée en ce que** l'un au moins des moyens de retenue (2) comprend au moins deux branches (5).

6. Sonde selon l'une des revendications précédentes, **caractérisée en ce que** chaque moyen de retenue comporte une partie formant tige (4) qui est reliée à l'élément filiforme (1) et qui est munie d'un trou borgne (6) apte à recevoir bâtonnet (7) permettant de la manoeuvrer.

7. Sonde selon l'une des revendications précédentes, **caractérisée en ce que** l'élément filiforme (1) a une longueur comprise entre environ 15 et 30 mm.

8. Sonde selon l'une des revendications précédentes, **caractérisée en ce que** l'élément filiforme (1) a une longueur comprise entre environ 38 et 60 mm.

## Claims

1. Bicanalicular probe for the treatment of an abnormality of lacrimation of the eye, this probe including a filiform element (1) made of a biologically inert material which, through introduction of the probe, is selectively inserted into the upper (11) and lower (13) lacrimal canaliculi of the eye and which has two free extremities (4) provided with respective terminal elements (2) selectively positioned in the lacrimal sac (15) or in the nasal fossa (62), **characterised in that** the terminal elements (2) consist of flexible retaining means which fold towards the filiform element (1) when the probe is introduced and open out in the lacrimal sac (15) or the nasal fossa (62) to form a retaining stop, and **in that** a play, allowing sliding movement of the probe positioned in the lacrimal canaliculi (11, 13), is provided between the retaining means (2) and the lacrimal sac (15) or the nasal fossa (62), this probe enabling lacrimation of the eye to be remedied by maintaining or re-establishing the flow of lacrimal fluid.

2. Probe according to claim 1, **characterised in that** at least one of the retaining means (2) consists of an anchor (3) with at least one flexible leg (5).

3. Probe according to claim 2, **characterised in that** each flexible leg (5) of the anchor (3), in the resting state, is inclined toward the filiform element (1).

4. Probe according to claim 1, **characterised in that** at least one of the retaining means (2) consists of a flexible disc.

5. Probe according to any one of claims 1 to 3, **characterised in that** at least one of the retaining means (2) has at least two legs (5).

6. Probe according to one of the preceding claims, **characterised in that** each retaining means has a part forming a shank (4) which is connected to the filiform element (1) and which is provided with a blind bore (6) capable of receiving a small rod (7) allowing it to be manoeuvred.

7. Probe according to one of the preceding claims, **characterised in that** the length of the filiform element (1) is between approximately 15 and 30 mm.

8. Probe according to one of the preceding claims, **characterised in that** length of the filiform element (1) is between approximately 38 and 60 mm.

## Patentansprüche

1. Zweikanalsonde zur Behandlung einer Tränensekretionsanomalie des Auges, wobei die Sonde ein fadenförmiges Element (1) aus biologischem inertem Material aufweist, das, durch Einführen der Sonde, selektiv in den oberen (11) und unteren (13) Tränenkanal des Auges eingeführt wird und das zwei freie Enden (4) aufweist, die jeweils mit terminalen Elementen (2) versehen sind, die selektiv in dem Tränensack (15) oder der Nasenhöhle (62) angeordnet sind, **dadurch gekennzeichnet, dass** die terminalen Elemente (2) aus flexiblen Rückhaltemitteln bestehen, die sich während der Einführung der Sonde in Richtung des fadenförmigen Elements (1) krümmen und sich in dem Tränensack (15) oder in der Nasenhöhle (62) ausdehnen, um Rückhaltestopper zu bilden, und **dadurch**, dass ein Spiel, das das Verschieben der in den Tränenkanälen (11, 13) oder der Nasenhöhle (62) angeordneten Sonde zulässt, zwischen den Rückhaltemitteln (2) und dem Tränensack (15) oder der Nasenhöhle (62) vorgesehen ist, wobei die Sonde die Heilung eines Tränens des Auges durch Aufrecherhalten oder Wiederherstellen des Tränenflüssigkeitsflusses gestattet.

2. Sonde nach Anspruch 1, die **dadurch gekennzeichnet ist, dass** das mindestens eine Rückhaltemittel (2) aus einem Anker (3) besteht, der mindestens einen biegsamen Arm (5) aufweist.

3. Sonde nach Anspruch 2, **dadurch gekennzeichnet, dass** jeder biegsame Arm (5) des Ankers (3) im Ruhezustand in Richtung des fadenförmigen Elementes (1) geneigt ist.

4. Sonde nach Anspruch 1, **dadurch gekennzeichnet, dass** das mindestens eine der Rückhaltemittel (2) aus einer biegsamen Scheibe besteht.

5. Sonde nach einem der Ansprüche 1 bis 3, **dadurch gekennzeichnet, dass** das mindestens eine der Rückhaltemittel (2) mindestens zwei Arme (5) aufweist.

6. Sonde nach einem der vorhergehenden Ansprüche, **dadurch gekennzeichnet, dass** jedes Rückhaltemittel einen Teil aufweist, der einen Schaft (4) bildet, der mit dem fadenförmigen Element verbunden und mit einem Blindloch (6) versehen ist, das dazu ausgelegt ist, das Stäbchen (7) aufzunehmen, das erlaubt, ihn zu bedienen.

7. Sonde nach einem der vorhergehenden Ansprüche, **dadurch gekennzeichnet, dass** das fadenförmige Element (1) eine Länge zwischen etwa 15 und 30 mm aufweist.

8. Sonde nach einem der vorhergehenden Ansprüche, **dadurch gekennzeichnet, dass** das fadenförmige Element (1) eine Länge zwischen etwa 38 und 60 mm aufweist.
